# EUROPEAN PATENT SPECIFICATION

(11) **EP 4 226 955 B1**
(45) Date of publication and mention of the grant of the patent: **11.02.2026**
(21) Application number: 21877254.9
(22) Date of filing: 25.08.2021
(51) Int. Cl.: A61L 27/56, A61F 2/28, A61L 27/12, A61L 27/58, B01D 39/20

(54) **ARTIFICIAL BONE, METHOD FOR MANUFACTURING THREE-DIMENSIONAL SHAPED OBJECT, AND CONTROL PROGRAM**
KÜNSTLICHER KNOCHEN, VERFAHREN ZUR HERSTELLUNG EINES DREIDIMENSIONALEN FORMOBJEKT UND STEUERUNGSPROGRAMM
OS ARTIFICIEL, PROCÉDÉ DE FABRICATION D'UN OBJET FAÇONNÉ TRIDIMENSIONNEL ET PROGRAMME DE COMMANDE

(30) Priority: 07.10.2020 JP 2020169561
(43) Date of publication of application: 16.08.2023
(73) Proprietor: RIKEN, Wako-shi, Saitama 351-0198 (JP)
(72) Inventor: YOKOTA Hideo, Wako-shi, Saitama 351-0198 (JP); YAMAZAWA Kenji, Wako-shi, Saitama 351-0198 (JP); TSUJIMURA Yuki, Wako-shi, Saitama 351-0198 (JP); OYAMA Shintaro, Wako-shi, Saitama 351-0198 (JP); WATANABE Masaki, Wako-shi, Saitama 351-0198 (JP)
(74) Representative: Grünecker Patent- und Rechtsanwälte PartG mbB
(86) International application number: PCT/JP2021/031070
(87) International publication number: WO 2022/074951

(56) References cited:
- CN-A- 104 529 505
- JP-A- 2004 224 658
- JP-A- 2007 501 054
- JP-A- 2010 531 694
- JP-A- 2012 223 373
- JP-A- H03 261 688
- JP-A- H04 305 075
- US-A1- 2015 259 247
- US-A1- 2017 304 056

## Description

### TECHNICAL FIELD

The present invention relates to a three-dimensional shaped object formed of a ceramic material, a method for manufacturing the same, and a control program.

### BACKGROUND ART

An artificial bone is a three-dimensional shaped object formed of a ceramic material. The artificial bone is charged in a missing portion of a bone caused by bone breaking or the like. The artificial bone formed of a bio-ceramic material has replaceability with a living bone, differently from the artificial bone formed of a metal material. In other words, the artificial bone using the bio-ceramic material can be fused and replaced with a natural bone by growth of a new bone after being charged in the missing portion of the bone in a living body.

Patent Literature 1 discloses the following. An artificial bone is formed by a powder lamination method using a powder bone material of a ceramic material such as calcium phosphate. Then, the artificial bone is immersed in an aqueous solution, and is subjected to depressurization processing. Thereby, a gas inside the artificial bone is replaced with an aqueous solution. The artificial bone is sequentially left still to cause hydration reaction to occur. Thus, a strength of the artificial bone is improved.

Patent Literature 2 discloses a layer stack object formation method that can form with a high strength and a good precision, a layer stack object that scarcely transforms to unabsorbable hydroxyapatite when transplanted into a living body, and has a high hardening speed, a high bone inducing ability, and a complex three-dimensional shape.

### Citation List

### Patent Literature

Patent Literature 1: Japanese Patent No. 4575295 Patent Literature 2: US 2015/259247 A1

### SUMMARY OF INVENTION

### TECHNICAL PROBLEM

Conventionally, a three-dimensional shaped object formed of a ceramic material needs to have an increased internal dense degree for the purpose of securing a strength or the like. However, in some cases, a low dense degree is preferable. In the case of an artificial bone as the three-dimensional shaped object, it is necessary to increase an internal dense degree in order to secure a strength of the artificial bone. However, a too high dense degree hinders growth of a new bone, thus resulting in that it takes time for the artificial bone to be fused with a living bone or to be replaced with the living bone. Meanwhile, a low dense degree of the artificial bone causes an insufficient strength for reinforcing the living bone.

In view of the above, a primary object of the present invention is to enable a three-dimensional shaped object to achieve both of a property in the case of a high dense degree and a property in the case of a low dense degree and to have a high strength.

A secondary object of the present invention is to enable an artificial bone to have at least one of fusibility and replaceability with a living bone and to have a high strength.

### SOLUTION TO PROBLEM

In order to achieve the above-described object, a three-dimensional shaped object includes: a dense portion formed on one side so as to be dense; and a porous portion formed on an opposite side so as to be porous, wherein the dense portion and the porous portion are formed by a reaction product of a ceramic material with a chelating agent. This three-dimensional shaped object may be an artificial bone.

A method according to the present invention is a method for manufacturing a three-dimensional shaped object (10), based on shape data representing a three-dimensional shape of a three-dimensional shaped object (10) and includes:
a layer forming step of forming a ceramic layer, using a powder ceramic material;
a curing liquid applying step of applying, to a target region in the layer, a curing liquid including a chelating agent; and
repeating the layer forming step and the curing liquid applying step so as to form the three-dimensional shaped object by a reaction product of the ceramic material with the chelating agent in the target regions of the layers superimposed on each other,
wherein the target region includes a one-side region and an opposite-side region that become a one-side portion and an opposite-side portion respectively in the three-dimensional shaped object, and

the curing liquid applying step includes applying the curing liquid to the target region, based on a position range of each of the one-side region and the opposite-side region expressed by the shape data, such that an amount of the curing liquid applied to each unit area in the one-side region is larger than an amount of the curing liquid applied to each unit area in the opposite-side region, thereby forming a dense portion (3) as the one-side portion, and forming a porous portion (5) as the opposite-side portion.

A program according to the present invention is a control program for controlling a three-dimensional shaping apparatus, based on shape data representing a three-dimensional shape of a three-dimensional shaped object (10), the control program causing the three-dimensional shaping apparatus (100) to execute:
layer forming processing of forming a ceramic layer, using a powder ceramic material;
curing liquid applying processing of applying to a target region in the layer, a curing liquid including a chelating agent; and
processing of repeating the layer forming processing and the curing liquid applying processing so as to form the three-dimensional shaped object by a reaction product of the ceramic material with the chelating agent in the target regions of the layers superimposed on each other,
wherein the target region in each of the layers includes a one-side region and an opposite-side region that constitute a one-side portion and an opposite-side portion respectively in the three-dimensional shaped object, and based on a position range of each of the one-side region and the opposite-side region expressed by the shape data,
the control program causes three-dimensional shaping apparatus (100) to perform the curing liquid applying processing such that an amount of the curing liquid applied to each unit area in the one-side region is larger than an amount of the curing liquid applied to each unit area in the opposite-side region, thereby forming a dense portion (3) as the one-side portion, and forming a porous portion (5) as the opposite-side portion.

### ADVANTAGEOUS EFFECTS OF INVENTION

The three-dimensional shaped object includes the dense portion having a high dense degree and the porous portion having a low dense degree, and for this reason, can achieve both of a property in the case of a high dense degree and a property in the case of a low dense degree. The porous portion is formed by the reaction product of the ceramic material with the chelating agent. Thus, even the porous portion can have a high strength.

### BRIEF DESCRIPTION OF DRAWINGS

FIG. 1 is a schematic configuration diagram illustrating one example of a three-dimensional shaped object according to the present embodiment.
FIG. 2 is a sectional view taken along the line II-II in FIG. 1.
FIG. 3 is a flowchart illustrating a method for manufacturing the three-dimensional shaped object according to the present embodiment.
FIG. 4A is an illustration of the method for manufacturing the three-dimensional shaped object.
FIG. 4B is another illustration of the method for manufacturing the three-dimensional shaped object.
FIG. 5 is a schematic configuration diagram illustrating another example of the three-dimensional shaped object according to the present embodiment.

### DESCRIPTION OF EMBODIMENTS

The following describes an embodiment of the present invention with reference to the drawings. The same reference sign is allocated to the corresponding part in each of the drawings, and duplicate description is omitted.

### (Configuration of Three-Dimensional Shaped Object)

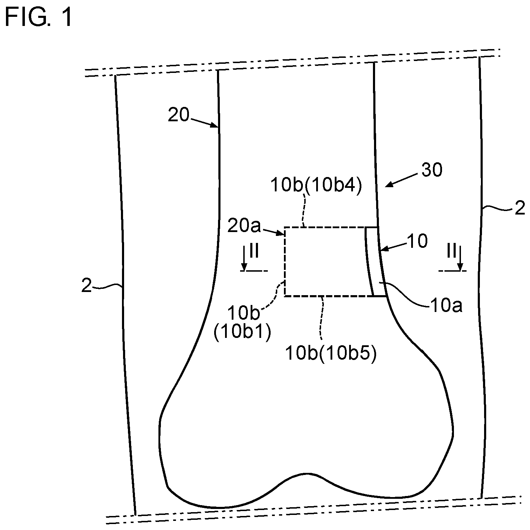

FIG. 1 is a diagram illustrating an example of a three-dimensional shaped object 10 according to the present embodiment. FIG. 1 illustrates the case where the three-dimensional shaped object 10 is an artificial bone, but the three-dimensional shaped object 10 is not limited to an artificial bone, and may be applied as a filter, a catalyst carrier, or the like as described below. Hereinafter, the description that concerns the three-dimensional shaped object 10 without specifying the artificial bone 10 applies to both of the case where the three-dimensional shaped object 10 is an artificial bone and the case where the three-dimensional shaped object 10 is not an artificial bone.

The artificial bone 10 is charged in a missing portion 20a of the living bone 20 in a living body (human or animal). FIG. 1 illustrates a state in which the artificial bone 10 is charged in the missing portion 20a of the living bone 20 on an inner side of a human skin 2. The missing portion 20a may be, for example, a portion caused by an injury (e.g., breaking of the bone) or a portion formed by osteotomy in an orthopedic surgery operation. The living bone 20 illustrated in FIG. 1 is a human thigh bone. However, the missing portion in which the artificial bone 10 according to the present embodiment can be charged may be a missing portion of a shinbone, a humerus bone, a thoracic bone, a skull, or a bone at another location in a human, or may be a missing portion of a living bone in an animal other than a human.

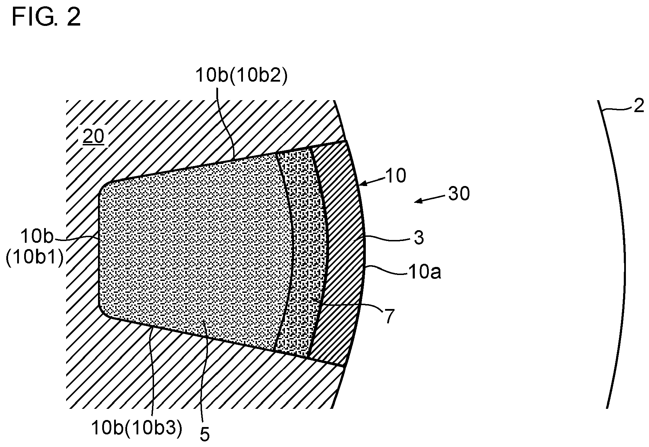

FIG. 2 is a sectional view taken along line II-II of the artificial bone 10 in FIG. 1. Each section of the artificial bone 10 in FIG. 1 parallel to the section in FIG. 2 may be the same section as that in FIG. 2.

The three-dimensional shaped object 10 has a dense portion 3 formed on one side (a right side in FIG. 2) so as to be dense, and a porous portion 5 formed on an opposite side (a left side in FIG. 2) so as to be porous. In the three-dimensional shaped object 10, the one side as the dense portion 3 and the opposite side as the porous portion 5 may be one end side and an opposite end side in the three-dimensional shaped object 10 (in the case of FIG. 2, the right side and the left side in the drawing). However, the present invention is not limited to this. For example, the one side as the dense portion 3 and the opposite side as the porous portion 5 may be an outer peripheral side and an inner central side (or an inner central side and an outer peripheral side) in the three-dimensional shaped object 10.

The dense portion 3 and the porous portion 5 are each formed by a reaction product of a ceramic material with a chelating agent. In other words, the ceramic material is cured as a result of chelating reaction with the chelating agent, and becomes the above-described reaction product. More specifically, the chelating agent dissolves the ceramic material, and calcium ions are thereby generated, and are trapped by the chelating agent so that the cured reaction product is obtained. In other words, the chelating reaction and solidification of the dissolved ceramic material result in the reaction product. The chelating reaction means chemical reaction in which a bi- or higher dentate ligand forms a ring and is bonded to central metal. The chelating agent means a compound having a ligand (polydentate ligand) that forms the above-described reaction product.

The dense portion 3 is a region where a dense degree is equal to or higher than a first threshold value. The porous portion 5 is a region where a dense degree is lower than a second threshold value. A dense degree indicates a ratio (occupancy percentage) of a space occupied by the constituent material (the reaction product) in the three-dimensional shaped object 10. Accordingly, a dense degree is 100% in a region in which the constituent material is completely charged and that includes no cavities. The first threshold value may be a value (e.g., 80%) within a range equal to or higher than 70% and is equal to or lower than 90%. The second threshold value may be a value (e.g., 60%) within a range equal to or higher than 30% and is equal to or lower than 65%. An upper limit of a dense degree of the dense portion 3 may be 100%. A lower limit of a dense degree of the porous portion 5 may be a value (e.g., 20%) within a range equal to or higher than 10% and is equal to or lower than 20%.

The three-dimensional shaped object 10 may include an intermediate portion 7 formed by the reaction product of the ceramic material with the chelating agent so as to be located between the dense portion 3 and the porous portion 5. A dense degree of the intermediate portion 7 is higher than a dense degree of the porous portion 5, and is lower than a dense degree of the dense portion 3. The intermediate portion 7 is bonded directly to each of the dense portion 3 and the porous portion 5. In one example, the intermediate portion 7 has a dense degree that gradually decreases as a position is more shifted from a side of the dense portion 3 to a side of the porous portion 5 (e.g., from a boundary with the dense portion 3 to a boundary with the porous portion 5). In another example, the intermediate portion 7 has a dense degree that is uniform over the entirety of the intermediate portion 7. When the intermediate portion 7 is not provided, the dense portion 3 and the porous portion 5 are bonded directly to each other.

The entirety of the three-dimensional shaped object 10 may be formed mainly of the above-described reaction product. For example, a ratio of a weight of the reaction product constituting the three-dimensional shaped object 10 to a total weight of the three-dimensional shaped object 10 may be equal to or higher than 80% (or equal to or higher than 90%) and equal to or lower than 100%. When the three-dimensional shaped object 10 is an artificial bone, the artificial bone 10 may further include one or both of the below-described cells and growth factors as components different from the above-described reaction product.

The above-described ceramic material may be calcium phosphate. The calcium phosphate may be, for example, α-tricalcium phosphate (α-TCP), β-tricalcium phosphate (β-TCP), octacalcium phosphate (OCP), or carbonate apatite, but is not limited to the specific calcium phosphate.

The above-described chelating agent may be an organic compound having three or more phosphate groups or carboxyl groups per one molecule. This organic compound may be etidronic acid, phytic acid, or citric acid, but is not limited to these.

The artificial bone 10 is charged in the missing portion 20a of the living bone 20 so as to form a bone body 30 together with the living bone 20. FIG. 1 illustrates one example of a state in which the bone body 30 is formed by the living bone 20 and the artificial bone 10 charged in the missing portion 20a. A state in which the artificial bone 10 is charged in the missing portion 20a of the living bone 20 is hereinafter referred to simply as a charged state.

The artificial bone 10 includes an outer surface 10a that is an outer surface of the bone body 30 in the charged state, and an inner surface 10b that is located inside the bone body 30 in the charged state. The outer surface of the bone body 30 is a surface facing to an outside of the living body (to a side of the skin 2). The outer surface 10a (e.g., the entirety of the outer surface 10a) is formed by the dense portion 3, and the porous portion 5 forms the inner surface 10b (a portion that is included in the inner surface 10b and that does not belong to the dense portion 3). In the charged state, the inner surface 10b formed by the porous portion 5 is adjacent to (e.g., in contact with) an inner surface of the missing portion 20a. The inner surface of the missing portion 20a is formed by the living bone 20.

As the inner surface 10b formed by the dense portion 3, a plurality of inner surfaces 10b may exist so as to face in mutually different directions. In the example of FIG. 1 and FIG. 2, the first to fifth inner surfaces 10b1 to 10b5 are formed as the inner surface 10b. The first inner surface 10b1 faces to a side (a left side in FIG. 2) opposite to the outer surface 10a, and is adjacent to (or in contact with) the bottom surface of the missing portion 20a among the inner surfaces that define the missing portion 20a. The second and third inner surfaces 10b2 and 10b3 are located between the first inner surface 10b1 and the outer surface 10a, and face to sides (an upper side and a lower side in FIG. 2) opposite to each other. The fourth and fifth inner surfaces 10b4 and 10b5 are located between the first inner surface 10b1 and the outer surface 10a while being located between the second inner surface 10b2 and the third inner surface 10b3, and face to sides (an upper side and a lower side in FIG. 1) opposite to each other.

The number of inner surfaces 10b is not limited to this example, and varies depending on a shape of the missing portion 20a in which the artificial bone 10 is charged. The three-dimensional shaped object (artificial bone) 10 may have a rectangular parallelepiped shape, a prismatic shape, a solid cylindrical shape, a pyramidal shape, a conical shape, a truncated conical shape, a truncated pyramidal shape, or a thin-piece shape. In the case of the artificial bone 10 having the prismatic shape or the solid cylindrical shape, one end surface of the prismatic shape or the solid cylindrical shape may be the outer surface 10a. In the case of the artificial bone 10 having the pyramidal shape, the conical shape, the truncated conical shape, or the truncated pyramidal shape, a bottom surface of the shape may be the outer surface 10a. In the case of the artificial bone 10 having the thin-piece shape, the artificial bone 10 may be charged in the missing portion 20a penetrating a skull (e.g., a calvarium) in a thickness direction thereof. In this case, the artificial bone 10 includes, as the above-described outer surface 10a, one side surface facing in its thickness direction.

The artificial bone 10 may include one or both of cells and growth factors. The cells and the growth factors, which are described in more detail below, are provided to promote replacement of the artificial bone 10 with natural bone tissue.

### (Method for Manufacturing Three-Dimensional Shaped Object)

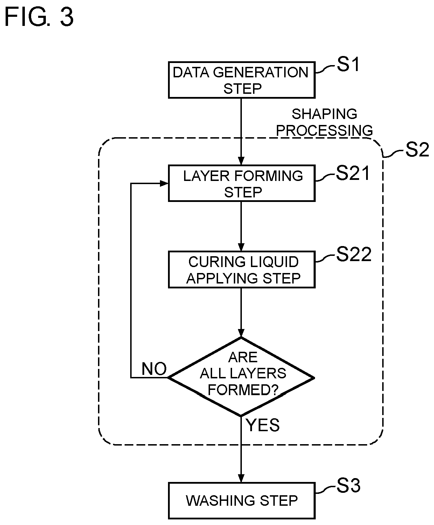

FIG. 3 is a flowchart illustrating a method for manufacturing the three-dimensional shaped object (artificial bone) 10 according to the embodiment of the present invention.

At a step S1, data (hereinafter, also referred to simply as shape data) for a three-dimensional shape of the three-dimensional shaped object 10 to be manufactured are generated. When the three-dimensional shaped object 10 is an artificial bone, the shape data representing the three-dimensional shape of the artificial bone 10 to be charged in the missing portion 20a are generated at the step S1, based on data of the three-dimensional shape of the missing portion 20a of the living bone 20. The shape data may include data representing a three-dimensional region occupied by the dense portion 3 that is a one-side portion of the three-dimensional shaped object 10. The shape data may include data representing a three-dimensional region occupied by the porous portion 5 that is an opposite-side portion of the three-dimensional shaped object 10. The shape data may include data representing a three-dimensional region occupied by the intermediate portion 7 that is a portion between the dense portion 3 and the porous portion 5 in the three-dimensional shaped object 10.

The shape data may be generated by a person operating an input device (such as a keyboard or a mouse) of a computer. When the three-dimensional shaped object 10 is the artificial bone 10, for example, data of the three-dimensional shape of the missing portion 20a may be displayed on a display of the computer, and a person may operate the input device while viewing the displayed data so that the shape data are generated. The data of the three-dimensional shape of the missing portion 20a are three-dimensional shape data of a region including the missing portion 20a in the living bone 20, and may be acquired by a CT scan, for example. The data of the three-dimensional shape of the missing portion 20a and the shape data may be CAD data, for example.

At a step S2, shaping processing for manufacturing the three-dimensional shaped object 10 is performed by a powder lamination method, based on the shape data generated at the step S1. In other words, at the step S2, a three-dimensional shaping apparatus 100 (3D printer) described below forms and cures a layer made of a powder ceramic material, based on the shape data, performs the same processing on an upper surface of the cured layer, and repeats the same processing on an upper surface of the newly cured layer, thereby forming the three-dimensional shaped artificial bone 10 including a large number of the cured and integrated layers. The above-described shape data include a large number of slice data pieces corresponding to a large number of the respective layers.

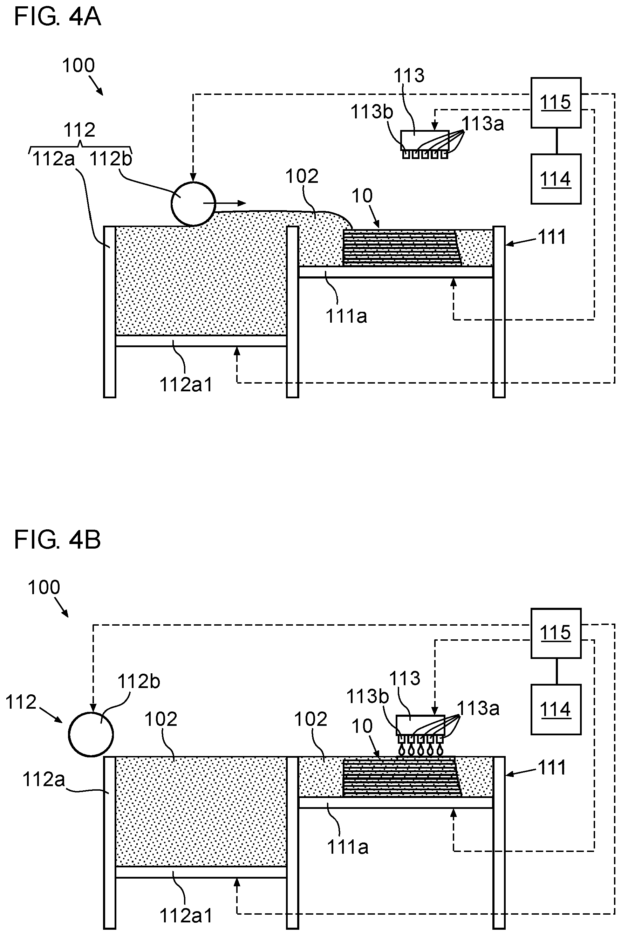

FIG. 4A is an illustration of the shaping processing, and depicts a configuration example of the three-dimensional shaping apparatus 100 used at the step S2. The three-dimensional shaping apparatus 100 of FIG. 4A manufactures the three-dimensional shaped object 10 by the powder lamination method. The three-dimensional shaping apparatus 100 includes a shaping container 111, a powder introduction device 112, and a curing device 113. The shaping container 111 includes an ascending-descending bottom portion 111a that forms a bottom portion of the shaping container 111, and ascends and descends. A powder ceramic material 2 (hereinafter, also referred to simply as powder 102) is introduced to the ascending-descending bottom portion 111a. The introduction device 112 introduces the powder 102 to the shaping container 111. The curing device 113 partially cures the introduced powder 102 in the shaping container 111. Further, a storage device 114 that stores the shape data generated at the above-described step S1, and a control device 115 that controls an operation of the three-dimensional shaping apparatus 100 are provided.

The step S2 includes a step S21 that is a layer forming step, and a step S22 that is a curing liquid applying step. At the step S2, the three-dimensional shaping apparatus 100 manufactures the three-dimensional shaped object 101 by repeating the steps S21 and S22 in this order as follows.

At the step S21, the powder 102 is used to form a ceramic layer. For example, as illustrated in FIG. 4A, the control device 115 controls the powder introduction device 112 such that the powder introduction device 112 introduces one layer of the powder 102 to the shaping container 111. At this time, the powder introduction device 112 introduces the powder 102 to the shaping container 111 such that an upper surface of the introduced powder 102 in the shaping container 111 becomes horizontal.

In the example of FIG. 4A, the powder introduction device 112 includes a powder container 112a and a roller 112b. The powder container 112a includes an ascending-descending bottom portion 112a1, and stores the powder 102. An upper surface of the powder container 112a is open. The control device 115 causes the ascending-descending bottom portion 112a1 to ascend by a distance corresponding to one layer. Next, as illustrated in FIG. 4A, the control device 115 moves the roller 112b horizontally from a side opposite to the shaping container 111 with respect to an opening of the powder container 112a so as to traverse the opening of the powder container 112a and an opening of the shaping container 111. As a result, one layer of the powder 102 that has ascended from the opening of the powder container 112a is introduced to the shaping container 111. In this method, the one layer of the powder 102 is introduced to the shaping container 111, and an upper surface of the introduced powder 102 is made horizontal.

At the step S22, curing liquid (hereinafter, also referred to simply as curing liquid) that is liquid including the chelating agent is applied to a target region on the upper surface of the layer formed at the step S21. For example, the control device 115 controls the curing device 113, based on the above-described slice data piece corresponding to the one layer, Thereby, the curing device 113 applies the curing liquid to the target region in the layer as illustrated in FIG. 4B. As a result, the powder ceramic material 102 in the target region becomes the cured reaction product as a result of the chelating reaction with the chelating agent included in the curing liquid.

At the step S22, an application amount of the curing liquid is controlled. In other words, the following is performed on the assumption that the target region in the layer formed at the step S21 includes a one-side region that comes to constitute a one-side portion of the three-dimensional shaped object 10, and an opposite-side region that comes to constitute an opposite-side portion of the three-dimensional shaped object 10. Based on the shape data, an amount of the curing liquid applied to each unit area in the one-side region by the curing device 113 may be controlled so as to be larger than an amount of the curing liquid applied to each unit area in the opposite-side region by the curing device 113. As a result, the one-side region is cured so as to constitute the dense portion 3, and the opposite-side region is cured so as to form the porous portion 5.

Further, in such control of an application amount of the curing liquid, the following is performed on the assumption that the target region in the layer formed at the step S21 includes an intermediate region between the one-side region and the opposite-side region. An amount of the curing liquid applied to each unit area in the intermediate region by the curing device 113 may be controlled so as to be smaller than the amount of the curing liquid applied to each unit area in the one-side region by the curing device 113 and be larger than the amount of the curing liquid applied to each unit area in the opposite-side region by the curing device 113. As a result, the intermediate region is cured so as to constitute the intermediate portion 7.

In addition, in the control of an application amount of the curing liquid, the amount of the curing liquid applied to each unit area at a position in the intermediate region by the curing device 113 may be changed depending on the position in the intermediate region. For example, as a position in the intermediate region is more shifted from a side of the one-side region to a side of the opposite-side region, the amount of the curing liquid applied to a unit area at the position by the curing device 113 may be gradually reduced. As a result, the intermediate portion 7 is formed so as to have a dense degree that gradually decreases as a position is more shifted from a side of the dense portion 3 to a side of the porous portion 5.

The above-described control of an application amount of the curing liquid may be performed by the control device 115, based on position ranges of the one-side region, the opposite-side region, and the intermediate region expressed by the shape data (slice data pieces).

When the step S22 is ended, the control device 115 causes the ascending-descending bottom portion 111a to descend by a thickness corresponding to one layer. Then, the step S21 and step S22 are performed again. Repeating the step S21 and step S22 in this manner forms the three-dimensional shaped object (artificial bone) 10 of the reaction product of the ceramic material with the chelating agent. In other words, the three-dimensional shaped object 10 is formed by forming and curing all the layers corresponding to all the slice data pieces of the shape data. After all the layers are formed and cured in this manner, the processing proceeds to a step S3.

At the step S3, the three-dimensional shaped object 10 formed at the step S2 is taken out from the shaping container 111, and the entire three-dimensional shaped object 10 is washed with liquid (e.g., water). For example, a water flow is supplied to the three-dimensional shaped object 10 so as to remove, from the three-dimensional shaped object 10, the residual chelating agent that does not contribute to the chelating reaction, and other components included in the curing liquid (e.g., the below-described surface active agent) .

Hereinafter, the shaping processing at the above-described step S2 is described in more detail.

### <Ceramic Material and Curing Liquid>

The powder ceramic material 102 used at the above-described step S21 may be calcium phosphate. The calcium phosphate may be any one of α-tricalcium phosphate (α-TCP), β-tricalcium phosphate (β-TCP), octacalcium phosphate (OCP), and carbonate apatite.

The curing liquid used at the step S22 is liquid including at least the chelating agent. The curing liquid may be obtained by dissolving the chelating agent in water. The chelating agent is an organic compound having three or more phosphate groups or carboxyl groups per one molecule. This organic compound may be any of etidronic acid, phytic acid, and citric acid.

The curing liquid may include another substance in addition to the chelating agent. For example, the curing liquid may further include a surface active agent.

### <Specific Example of Three-Dimensional Shaping Apparatus>

The three-dimensional shaping apparatus 100 may be one that uses an inkjet printer. In other words, the above-described curing device 113 of the three-dimensional shaping apparatus 100 may be a constituent element of the inkjet printer. In this case, the curing liquid is used as ink of the inkjet printer or instead of the ink. In this case, the curing device 113 is a movable carriage provided with a plurality of heads 113a for dropping or ejecting the curing liquid.

At the above-described step S22, the control device 115 controls the carriage 113 so as to be moved along the target region above the target region of the layer, and controls the heads 113a so as to drop or eject the curing liquid to respective positions in the target region. As a result, the curing liquid is applied to the entire target region in the layer.

In this case, in the control of an application amount of the curing liquid at the step S22, the control device 115 may control the application amount of the curing liquid by causing the number of the heads 113a used for applying the curing liquid to each unit area in the above-described one-side region to be larger than the number of the heads 113a used for applying the curing liquid to each unit area in the above-described opposite-side region.

In addition, similarly, for the above-described intermediate region, the control device 115 may control the application amount of the curing liquid by causing the number of the heads 113a used for applying the curing liquid to each unit area in the intermediate region to be smaller than the number of the heads 113a used for applying the curing liquid to each unit area in the above-described one-side region and to be larger than the number of the heads 113a used for applying the curing liquid to each unit area in the above-described opposite-side region. In this case, as a position in the intermediate region is more shifted from a side of the one-side region to a side of the opposite-side region, the control device 115 may gradually reduce the number of heads 113a used for applying the curing liquid to each unit area at the position. As a result, the intermediate portion 7 may be formed so as to have a dense degree that gradually decreases as a position is more shifted from a side of the dense portion 3 to a side of the porous portion 5.

### <Cells and Growth Factors>

In the case of the three-dimensional shaped object 10 as an artificial bone, for the purpose of promoting replacement of the artificial bone 10 with natural bone tissue of the living bone 20 after charging the artificial bone 10 in the living bone 20, one or both of cells and growth factors may be applied to the entirety or a predetermined portion of the target region in the layer at the step S22. The cells may be cells (e.g., osteoblasts and osteoclasts) existing in the bone tissue, may be stem cells before differentiation, or may be one or both thereof. The growth factors may be bone morphogenetic protein (BMP) having strong ability to induce bone regeneration, or may be a basic fibroblast growth factor (b-FGF), a transforming growth factor (TGFβ), an insulin-like growth factor (IGF-1), or the like as cytokine that promotes bone cell growth or matrix growth.

In the case where the cells are applied to the artificial bone 10, a cell inclusion liquid including the cells is prepared. For example, the cell inclusion liquid may be a liquid in which cells are dispersed. At the above-described step S22, the cell inclusion liquid is dropped or ejected to the entirety or a predetermined region of the target region of the layer from another head 113b provided in the above-described carriage 113. This predetermined region may be, for example, the opposite-side region that becomes the porous portion 5.

In the case where the growth factors as well as the cells are applied to the artificial bone 10, the growth factors may be included in the above-described cell inclusion liquid, or a growth factor inclusion liquid including the growth factors may be prepared in addition to the above-described cell inclusion liquid. In the latter case, at the above-described step S22, the growth factor inclusion liquid is dropped or ejected to the entirety or a predetermined region (e.g., the opposite-side region) of the target region of the layer from still another head (not illustrated) provided in the carriage 113. In the case where only the growth factors out of the cells and the growth factors are applied to the artificial bone 10, at the step S22, the growth factor inclusion liquid is dropped or ejected to the entirety or a predetermined region (e.g., the opposite-side region) of the target region of the layer from the head 113b provided in the carriage 113.

### (Advantageous Effects of Embodiment)

The above-described three-dimensional shaped object 10 includes the dense portion 3 having a high dense degree and the porous portion 5 having a low dense degree, and for this reason, can achieve both of a property in the case of the high dense degree and a property in the case of the low dense degree.

The porous portion 5 is formed by the reaction product of the ceramic material with the chelating agent. Thus, even the porous portion 5 can have a high strength. For example, in the case of the three-dimensional shaped object 10 as the artificial bone, the porous portion 5 can have a strength of 25 MPa to 30 MPa even when the dense degree is 60%.

In the case of the three-dimensional shaped object 10 as the artificial bone, the dense portion 3 having a strength higher than that of the porous portion 5 forms the outer surface of the bone body 30 in a charged state where the artificial bone 10 is charged in the missing portion 20a of the living bone 20 such that the artificial bone 10 and the living bone 20 form the bone body 30. Accordingly, the dense portion 3 can reliably protect the missing portion 20a (i.e., the artificial bone 10 charged in the missing portion 20a) against impact from an outside.

The inner surface 10b formed by the porous portion 5 is adjacent to (e.g., in contact with) the inner surface of the missing portion 20a of the living bone 20, in the above-described charged state. For this reason, new bone tissue (bone substance) easily enters the artificial bone 10 from the living bone 20 through the inner surface of the missing portion 20a. Thus, it is possible to implement the artificial bone 10 having high fusibility and replaceability with the living bone 20.

At the step S22 in the above-described shaping processing, the dense portion 3 and the porous portion 5 can be easily formed simply by changing an amount of the curing liquid applied to the layer of the powder 102. Further, the three-dimensional shaped object 10 is formed by the reaction product of the ceramic material with the chelating agent, and the reaction product itself has a high strength. Thus, it becomes unnecessary to perform firing treatment or the like for enhancing a strength.

The intermediate portion 7 may be provided between the dense portion 3 and the porous portion 5. In this case, the intermediate portion 7 has a dense degree that gradually decreases as a position is more shifted from a side of the dense portion 3 to a side of the porous portion 5. Thus, it is possible to enhance a strength of the three-dimensional shaped object 10.

### (Control Program)

A control program for performing the shaping processing at the above-described step S2 may be stored in the storage device 114, or may be stored in another storage device readable by a computer as the control device 115.

The control program causes the computer 115 to perform a layer forming processing. The layer forming processing causes the three-dimensional shaping apparatus 100 to form a ceramic layer, using the powder ceramic material 102. The layer forming processing is processing of causing the three-dimensional shaping apparatus 100 to perform the above-described step S21.

The control program causes the computer 115 to perform a curing liquid applying processing. The curing liquid applying processing causes the three-dimensional shaping apparatus 100 to apply, to the target region in the layer, the curing liquid including the chelating agent. The curing liquid applying processing is processing of causing the three-dimensional shaping apparatus 100 to perform the above-described step S22.

The control program causes the computer 115 to repeatedly perform the layer forming processing and the curing liquid applying processing. Thereby, the computer 115 causes the three-dimensional shaping apparatus 100 to repeatedly perform the step S21 and the step S22.

### (Other Examples of Three-Dimensional Shaped Object)

The above-described three-dimensional shaped object 10 may be an object other than the artificial bone. For example, the three-dimensional shaped object 10 may be a filter that collects particles in passing fluid.

The filter 10 may be, for example, an exhaust gas purification filter, an air cleaning filter, or a liquid filter. The exhaust gas purification filter is provided in an exhaust flow path of a heat engine such as an internal combustion engine or a boiler, and collects particles included in an exhaust gas from the heat engine. The air cleaning filter is provided in a flow path for circulating air in a room of a building, a vehicle, or the like, and collects particles in the circulating air. The liquid filter collects particles (foreign substances) in a liquid such as fuel or hydraulic oil.

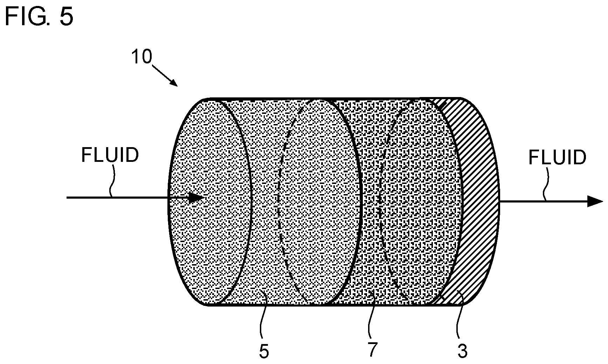

FIG. 5 is a schematic configuration diagram of the filter as the three-dimensional shaped object 10. Fluid can pass through the dense portion 3 and the porous portion 5 in the filter 10. The porous portion 5 constitutes a filter portion on an upstream side of the passing fluid, and the dense portion 3 constitutes a filter portion on a downstream side of the passing fluid. In the case where the intermediate portion 7 is provided in the filter 10 as illustrated in FIG. 5, the fluid can pass through the intermediate portion 7.

In such a configuration, the fluid flows into the filter 10 from the porous portion 5, passes through the porous portion 5, subsequently passes through the intermediate portion 7, then passes through the dense portion 3, and flows out of the filter 10. In this process, particles in the fluid are collected by the filter 10. Among the particles in the fluid, the large particles are collected in the porous portion 5, the particles having an intermediate size are collected in the intermediate portion 7, and the small particles are collected in the dense portion 3.

After the filter 10 is used for a predetermined period of time, for example, pressurized water may be made to flow through the filter 10 from a side of the dense portion 3 to a side of the porous portion 5. Thereby, the particles accumulated in the filter 10 are removed, and the filter 10 may be used again.

Alternatively, the three-dimensional shaped object 10 may be a catalyst carrier. In this case, the catalyst carrier 10 may have the same configuration (configuration in FIG. 5) as that of the above-described filter. The catalyst carrier 10 carries a catalyst in its inside (e.g., the porous portion 5, the intermediate portion 7, and the dense portion 3 in FIG. 5). The catalyst carrier 10 promotes predetermined chemical reaction of the fluid (liquid or gas) passing through an inside thereof. In the case of the example of FIG. 5, the fluid flows into the catalyst carrier 10 from the porous portion 5, passes through the porous portion 5, subsequently passes through the intermediate portion 7, then passes through the dense portion 3, and flows out of the catalyst carrier 10. Alternatively, in the order reversed from this, the fluid may pass through the catalyst carrier 10.

In the example of FIG. 5, each of the porous portion 5, the dense portion 3, and the intermediate portion 7 has a solid cylindrical shape, but may have any of other shapes. In the example of FIG. 5, an entire shape of the filter 10 or the catalyst carrier 10 is a solid cylindrical shape, but may be any of other shapes such as a prismatic shape. In the filter 10 or the catalyst carrier 10, the porous portion 5 may have a solid cylindrical shape, the intermediate portion 7 may have a hollow cylindrical shape and be provided so as to surround an outer peripheral surface of the porous portion 5, and the dense portion 3 may have a hollow cylindrical shape and be provided so as to surround an outer peripheral surface of the intermediate portion 7. In this case, the fluid may flow into the porous portion 5 in an axial direction thereof, and then may pass through the intermediate portion 7 and the dense portion 3 in this order in a radial direction thereof.

In the case of the catalyst carrier 10, the dense portion 3 may have a solid cylindrical shape, the intermediate portion 7 may have a hollow cylindrical shape and be provided so as to surround an outer peripheral surface of the dense portion 3, and the porous portion 5 may have a hollow cylindrical shape and be provided so as to surround an outer peripheral surface of the intermediate portion 7. In this case, the fluid may flow into the dense portion 3 in an axial direction thereof, and then may pass through the intermediate portion 7 and the porous portion 5 in this order in a radial direction thereof.

### Reference Signs List

2 skin, 3 dense portion, 5 porous portion, 7 intermediate portion, 10 three-dimensional shaped object (artificial bone), 10a outer surface, 10b inner surface, 20 living bone, 20a missing portion, 30 bone body, 100 three-dimensional shaping apparatus, 102 powder ceramic material (powder), 111 shaping container, 111a ascending-descending bottom portion, 112 powder introduction device, 112a powder container, 112a1 ascending-descending bottom portion, 112b roller, 113 curing device (carriage), 113a head, 113b head, 114 storage device, 115 control device (computer)

## Claims

1. A control device (115) that controls a three-dimensional shaping apparatus (100), based on shape data representing a three-dimensional shape of a three-dimensional shaped object (10), the control device (115) causing the three-dimensional shaping apparatus (100) to perform:
layer forming processing of forming a ceramic layer, using a powder ceramic material;
curing liquid applying processing of applying, to a target region in the layer, a curing liquid including a chelating agent; and
repeating the layer forming processing and the curing liquid applying processing to cause the three-dimensional shaping apparatus (100) to form the three-dimensional shaped object (10) by a reaction product of the ceramic material with the chelating agent in the target regions of the layers superimposed on each other,
wherein the target region in each of the layers includes a one-side region and an opposite-side region that constitute a one-side portion and an opposite-side portion respectively in the three-dimensional shaped object (10), and
based on a position range of each of the one-side region and the opposite-side region expressed by the shape data, the control device (115) causes the three-dimensional shaping apparatus (100) to perform the curing liquid applying processing such that an amount of the curing liquid applied to each unit area in the one-side region is larger than an amount of the curing liquid applied to each unit area in the opposite-side region, thereby forming a dense portion (3) as the one-side portion, and forming a porous portion (5) as the opposite-side portion, and
wherein the dense portion and the porous portion are formed by a reaction product of a ceramic material with a chelating agent.

2. The control device (115) according to claim 1,
wherein the target region in each of the layers includes an intermediate region that constitutes an intermediate portion (7) between the dense portion (3) and the porous portion (5) in the three-dimensional shaped object (10), and
based on a position range of each of the one-side region, the opposite-side region, and the intermediate region expressed by the shape data, the control device (115) causes the three-dimensional shaping apparatus (100) to perform the curing liquid applying processing such that an amount of the curing liquid applied to each unit area in the intermediate region is smaller than an amount of the curing liquid applied to each unit area in the one-side region and is larger than an amount of the curing liquid applied to each unit area in the opposite-side region, thereby forming the intermediate portion (7) having a dense degree higher that a dense degree of the porous portion (5) and lower than a dense degree of the dense portion (3), and
wherein the intermediate portion has a dense degree higher than a dense degree of the porous portion and lower than a dense degree of the dense portion.

3. The control device according to claim 1 or 2,
wherein the dense portion (3) has a dense degree equal to or higher than 80%, and the porous portion (5) has a dense degree lower than 60%.

4. The control device (115) according to any one of claims 1 to 3,
wherein the ceramic material is calcium phosphate.

5. The control device (115) according to claim 4,
wherein the calcium phosphate is any one of α-tricalcium phosphate (α-TCP), β-tricalcium phosphate (β-TCP), octacalcium phosphate (OCP), and carbonate apatite.

6. The control device (115) according to claim 4 or 5,
wherein the chelating agent is an organic compound having three or more phosphate groups or carboxyl groups per one molecule.

7. The control device (115) according to claim 6,
wherein the organic compound is any one of etidronic acid, phytic acid, and citric acid.

8. The control device (115) according to claim 1,
wherein the three-dimensional shaped object (10) is an artificial bone to be charged in a missing portion (20a) of a living bone (20) so as to form a bone body (30) together with the living bone (20),
the artificial bone includes an outer surface (10a) serving as an outer surface of the bone body, and an inner surface (10b) to be located inside the bone body (30), and
the outer surface (10a) is formed by the dense portion (3), and the porous portion (5) forms the inner surface (10b).

9. The control device (115) according to claim 8,
wherein the inner surface (10b) formed by the porous portion (5) is a surface to be adjacent to an inner surface included in the missing portion (20a) and formed by the living bone (20).

10. The control device (115) according to claim 1 or 9,
wherein the curing liquid applying processing further includes:
causing the three-dimensional shaping apparatus (100) to drop or eject a cell inclusion liquid or a growth factor inclusion liquid to entirety or a predetermined region of the target region in each of the layers, wherein the cell inclusion liquid includes cells and optionally growth factors, and the growth factor inclusion liquid includes growth factors.

11. A method for manufacturing a three-dimensional shaped object (10), based on shape data representing a three-dimensional shape of a three-dimensional shaped object (10), the method comprising:
a layer forming step of forming a ceramic layer, using a powder ceramic material;
a curing liquid applying step of applying, to a target region in the layer, a curing liquid including a chelating agent; and
repeating the layer forming step and the curing liquid applying step so as to form the three-dimensional shaped object (10) by a reaction product of the ceramic material with the chelating agent in the target regions of the layers superimposed on each other,
wherein the target region in each of the layers includes a one-side region and an opposite-side region that constitute a one-side portion and an opposite-side portion respectively in the three-dimensional shaped object (10), and
the curing liquid applying step includes applying the curing liquid to the target region, based on a position range of each of the one-side region and the opposite-side region expressed by the shape data, such that an amount of the curing liquid applied to each unit area in the one-side region is larger than an amount of the curing liquid applied to each unit area in the opposite-side region, thereby forming a dense portion (3) as the one-side portion, and forming a porous portion (5) as the opposite-side portion.

12. The method for manufacturing the three-dimensional shaped object (10) according to claim 11,
wherein the target region in each of the layers includes an intermediate region between the one-side region and the opposite-side region, and
the curing liquid applying step includes applying the curing liquid to the target region, based on a position range of each of the one-side region, the opposite-side region, and the intermediate region expressed by the shape data, such that an amount of the curing liquid applied to each unit area in the intermediate region is smaller than the amount of the curing liquid applied to each unit area in the one-side region, and is larger than the amount of the curing liquid applied to each unit area in the opposite-side region, thereby forming an intermediate portion (7) having a dense degree higher that a dense degree of the porous portion (5) and lower than a dense degree of the dense portion (3).

13. The method for manufacturing a three-dimensional shaped object (10) according to claim 11 or 12, further comprising:
a washing step of washing the three-dimensional shaped object (10) after forming the three-dimensional shaped object (10).

14. A control program for controlling a three-dimensional shaping apparatus (100), based on shape data representing a three-dimensional shape of a three-dimensional shaped object (10), the control program causing the three-dimensional shaping apparatus (100) to execute:
layer forming processing of forming a ceramic layer, using a powder ceramic material;
curing liquid applying processing of applying, to a target region in the layer, a curing liquid including a chelating agent; and
processing of repeating the layer forming processing and the curing liquid applying processing so as to form the three-dimensional shaped object (10) by a reaction product of the ceramic material with the chelating agent in the target regions of the layers superimposed on each other,
wherein the target region in each of the layers includes a one-side region and an opposite-side region that constitute a one-side portion and an opposite-side portion respectively in the three-dimensional shaped object (10), and
based on a position range of each of the one-side region and the opposite-side region expressed by the shape data, the control program causes the three-dimensional shaping apparatus (100) to perform the curing liquid applying processing such that an amount of the curing liquid applied to each unit area in the one-side region is larger than an amount of the curing liquid applied to each unit area in the opposite-side region, thereby forming a dense portion (3) as the one-side portion, and forming a porous portion (5) as the opposite-side portion.

## Patentansprüche

1. Steuerungsvorrichtung (115), die eine Einrichtung (100) für dreidimensionales Formen auf Basis von Form-Daten steuert, die eine dreidimensionale Form eines dreidimensionalen geformten Objekts (10) repräsentieren, wobei die Steuerungsvorrichtung (115) die Einrichtung (100) für dreidimensionales Formen veranlasst, durchzuführen:
Schichtausbildungs-Verarbeitung zum Ausbilden einer Keramikschicht unter Verwendung eines pulverförmigen Keramikmaterials;
Verarbeitung zum Aufbringen von Aushärt-Flüssigkeit, mit der auf einen Zielbereich in der Schicht eine Aushärt-Flüssigkeit aufgebracht wird, die einen Chelatbildner enthält; sowie
Wiederholen der Schichtausbildungs-Verarbeitung und der Verarbeitung zum Aufbringen von Aushärt-Flüssigkeit, mit dem die Einrichtung für dreidimensionales Formen (10) veranlasst wird, das dreidimensionale geformte Objekt (10) mit einem Reaktionsprodukt des keramischen Materials mit dem Chelatbildner in den Zielbereichen der übereinander angeordneten Schichten auszubilden,
wobei der Zielbereich in jeder der Schichten einen Bereich einer Seite sowie einen Bereich einer gegenüberliegenden Seite einschließt, die einen Abschnitt einer Seite bzw. einen Abschnitt einer gegenüberliegenden Seite in dem dreidimensionalen geformten Objekt (10) bilden, und
die Steuerungsvorrichtung (115) auf Basis eines jeweiligen Positionsgebietes des Bereiches einer Seite und des Bereiches einer gegenüberliegenden Seite, ausgedrückt durch die Form-Daten, die Einrichtung (100) für dreidimensionales Formen veranlasst, die Verarbeitung zum Aufbringen von Aushärt-Flüssigkeit so durchzuführen, dass eine Menge der auf jede Flächeneinheit in dem Bereich einer Seite aufgebrachten Aushärt-Flüssigkeit größer ist als eine Menge der auf jede Flächeneinheit in dem Bereich einer gegenüberliegenden Seite aufgebrachten Aushärt-Flüssigkeit, so dass ein dichter Abschnitt (3) als der Abschnitt einer Seite ausgebildet wird und ein poröser Abschnitt (5) als der Abschnitt einer gegenüberliegenden Seite ausgebildet wird, und
wobei der dichte Abschnitt und der poröse Abschnitt durch ein Reaktionsprodukt eines keramischen Materials mit einem Chelatbildner ausgebildet werden.

2. Steuerungsvorrichtung (115) nach Anspruch 1,
wobei der Zielbereich in jeder der Schichten einen Zwischenbereich einschließt, der einen Zwischenabschnitt (7) zwischen dem dichten Abschnitt (3) und dem porösen Abschnitt (5) in dem dreidimensionalen geformten Objekt (10) bildet, und
die Steuerungsvorrichtung (115) auf Basis eines jeweiligen Positionsgebietes des Bereiches einer Seite, des Bereiches einer gegenüberliegenden Seite sowie des Zwischenbereiches, ausgedrückt durch die Form-Daten, die Einrichtung (100) für dreidimensionales Formen veranlasst, die Verarbeitung zum Aufbringen von Aushärt-Flüssigkeit so durchzuführen, dass eine Menge der auf jede Flächeneinheit in dem Zwischenbereich aufgebrachten Aushärt-Flüssigkeit kleiner ist als eine Menge der auf jede Flächeneinheit in dem Bereich einer Seite aufgebrachten Aushärt-Flüssigkeit und größer ist als eine Menge der auf jede Flächeneinheit in dem Bereich einer gegenüberliegenden Seite aufgebrachten Aushärt-Flüssigkeit, so dass der Zwischenabschnitt (7) ausgebildet wird, der einen Dichtegrad hat, der höher ist als ein Dichtegrad des porösen Abschnitts (5) und niedriger als ein Dichtegrad des dichten Abschnitts (3), und
wobei der Zwischenabschnitt einen Dichtegrad hat, der höher ist als ein Dichtegrad des porösen Abschnitts und niedriger als ein Dichtegrad des dichten Abschnitts.

3. Steuerungsvorrichtung nach Anspruch 1 oder 2,
wobei der dichte Abschnitt (3) einen Dichtegrad von 80 % oder höher hat und der poröse Abschnitt (5) einen Dichtegrad unter 60 % hat.

4. Steuerungsvorrichtung (115) nach einem der Ansprüche 1 bis 3,
wobei das Keramikmaterial Calciumphosphat ist.

5. Steuerungsvorrichtung (115) nach Anspruch 4,
wobei das Calciumphosphat eine beliebige Verbindung von α-Tricalciumphosphat (α-TCP), β-Tricalciumphosphat (β-TCP), Octacalciumphosphat (OCP) und Carbonatapatit ist.

6. Steuerungsvorrichtung (115) nach Anspruch 4 oder 5,
wobei der Chelatbildner eine organische Verbindung mit drei oder mehr Phosphatgruppen oder Carboxylgruppen pro ein Molekül ist.

7. Steuerungsvorrichtung (115) nach Anspruch 6,
wobei die organische Verbindung eine beliebige Verbindung von Etidronsäure, Phytinsäure und Zitronensäure ist.

8. Steuerungsvorrichtung (115) nach Anspruch 1,
wobei das dreidimensionale geformte Objekt (10) ein künstlicher Knochen ist, der in einen fehlenden Abschnitt (20a) eines lebenden Knochens (20) eingesetzt werden soll, um zusammen mit dem lebenden Knochen (20) einen Knochenkörper (30) zu bilden,
der künstliche Knochen eine Außenfläche (10a), die als eine Außenfläche des Knochenkörpers dient, sowie eine Innenfläche (10b) einschließt, die im Inneren des Knochenkörpers (30) positioniert sein soll, und
die Außenfläche (10a) durch den dichten Abschnitt (3) gebildet wird und der poröse Abschnitt (5) die Innenfläche (10b) bildet.

9. Steuerungsvorrichtung (115) nach Anspruch 8,
wobei die durch den porösen Abschnitt (5) gebildete Innenfläche (10b) eine Fläche ist, die an eine Innenfläche angrenzen soll, die in dem fehlenden Abschnitt (20a) eingeschlossen ist und durch den lebenden Knochen (20) gebildet wird.

10. Steuerungsvorrichtung (115) nach Anspruch 1 oder 9,
wobei die Schichtausbildungs-Verarbeitung des Weiteren einschließt:
Veranlassen, dass die Einrichtung (100) für dreidimensionales Formen eine Zelleinschluss-Flüssigkeit oder eine Wachstumsfaktoreinschluss-Flüssigkeit auf den gesamten Zielbereich in jeder der Schichten oder einen vorgegebenen Bereich desselben fallen lässt oder ausstößt, wobei die Zelleinschluss-Flüssigkeit Zellen und optional Wachstumsfaktoren enthält, und die Wachstumsfaktoreinschluss-Flüssigkeit Wachstumsfaktoren enthält.

11. Verfahren zum Herstellen eines dreidimensionalen geformten Objekts (10) auf Basis von Form-Daten, die eine dreidimensionale Form eines dreidimensionalen geformten Objekts (10) repräsentieren, wobei das Verfahren umfasst:
einen Schichtausbildungs-Schritt, in dem eine Keramikschicht unter Verwendung eines pulverförmigen keramischen Materials ausgebildet wird;
einen Schritt zum Aufbringen von Aushärt-Flüssigkeit, in dem auf einen Zielbereich in der Schicht eine Aushärt-Flüssigkeit aufgebracht wird, die einen Chelatbildner enthält; und
Wiederholen des Schichtausbildungs-Schrittes und des Schrittes zum Aufbringen von Aushärt-Flüssigkeit, um das dreidimensionale Objekt (10) mit einem Reaktionsprodukt des keramischen Materials mit dem Chelatbildner in den Zielbereichen der übereinander angeordneten Schichten auszubilden,
wobei der Zielbereich in jeder der Schichten einen Bereich einer Seite sowie einen Bereich einer gegenüberliegenden Seite einschließt, die einen Abschnitt einer Seite bzw. einen Abschnitt einer gegenüberliegenden Seite in dem dreidimensionalen geformten Objekt (10) bilden, und
der Schritt zum Aufbringen von Aushärt-Flüssigkeit einschließt, dass die Aushärt-Flüssigkeit auf den Zielbereich auf Basis eines jeweiligen Positionsgebietes des Bereiches einer Seite und des Bereiches einer gegenüberliegenden Seite, ausgedrückt durch die Form-Daten, so aufgebracht wird, dass eine Menge der auf jede Flächeneinheit in dem Bereich einer Seite aufgebrachten Aushärt-Flüssigkeit größer ist als eine Menge der auf jede Flächeneinheit in dem Bereich einer gegenüberliegenden Seite aufgebrachten Aushärt-Flüssigkeit, so dass ein dichter Abschnitt (3) als der Abschnitt einer Seite ausgebildet wird und ein poröser Abschnitt (5) als der Abschnitt einer gegenüberliegenden Seite ausgebildet wird.

12. Verfahren zum Herstellen des dreidimensionalen geformten Objekts (10) nach Anspruch 11,
wobei der Zielbereich in jeder der Schichten einen Zwischenbereich zwischen dem Bereich einer Seite und dem Bereich einer gegenüberliegenden Seite einschließt, und
der Schritt zum Aufbringen von Aushärt-Flüssigkeit einschließt, dass die Aushärt-Flüssigkeit auf Basis eines jeweiligen Positionsgebietes des Bereiches einer Seite, des Bereiches einer gegenüberliegenden Seite sowie des Zwischenbereiches, ausgedrückt durch die Form-Daten, so aufgebracht wird, dass eine Menge der auf jede Flächeneinheit in dem Zwischenbereich aufgebrachten Aushärt-Flüssigkeit kleiner ist als die Menge der auf jede Flächeneinheit in dem Bereich einer Seite aufgebrachten Aushärt-Flüssigkeit und größer ist als die Menge der auf jede Flächeneinheit in dem Bereich einer gegenüberliegenden Seite aufgebrachten Aushärt-Flüssigkeit, so dass ein Zwischenabschnitt (7) ausgebildet wird, der einen Dichtegrad hat, der höher ist als ein Dichtegrad des porösen Abschnitts (5) und niedriger als ein Dichtegrad des dichten Abschnitts (3).

13. Verfahren zum Herstellen eines dreidimensionalen geformten Objekts (10) nach Anspruch 11 oder 12, das des Weiteren umfasst:
einen Spülschritt, in dem das dreidimensionale geformte Objekt (10) nach Ausbilden des dreidimensionalen geformten Objekts (10) gespült wird.

14. Steuerungsprogramm , mit dem eine Einrichtung (100) für dreidimensionales Formen auf Basis von Form-Daten gesteuert wird, die eine dreidimensionale Form eines dreidimensionalen geformten Objekts (10) repräsentieren, wobei das Steuerungsprogramm die Einrichtung (100) für dreidimensionales Formen veranlasst, durchzuführen:
Schichtausbildungs-Verarbeitung zum Ausbilden einer Keramikschicht unter Verwendung eines pulverförmigen Keramikmaterials;
Verarbeitung zum Aufbringen von Aushärt-Flüssigkeit, mit der auf einen Zielbereich in der Schicht eine Aushärt-Flüssigkeit aufgebracht wird, die einen Chelatbildner enthält; sowie
Verarbeitung zum Wiederholen der Schichtausbildungs-Verarbeitung und der Verarbeitung zum Aufbringen von Aushärt-Flüssigkeit, um das dreidimensionale Objekt (10) mit einem Reaktionsprodukt des keramischen Materials mit dem Chelatbildner in den Zielbereichen der übereinander angeordneten Schichten auszubilden,
wobei der Zielbereich in jeder der Schichten einen Bereich einer Seite sowie einen Bereich einer gegenüberliegenden Seite einschließt, die einen Abschnitt einer Seite bzw. einen Abschnitt einer gegenüberliegenden Seite in dem dreidimensionalen geformten Objekt (10) bilden, und
das Steuerungsprogramm auf Basis eines jeweiligen Positionsgebietes des Bereiches einer Seite und des Bereiches einer gegenüberliegenden Seite, ausgedrückt durch die Form-Daten, die Einrichtung (100) für dreidimensionales Formen veranlasst, die Verarbeitung zum Aufbringen von Aushärt-Flüssigkeit so durchzuführen, dass eine Menge der auf jede Flächeneinheit in dem Bereich einer Seite aufgebrachten Aushärt-Flüssigkeit größer ist als eine Menge der auf jede Flächeneinheit in dem Bereich einer gegenüberliegenden Seite aufgebrachten Aushärt-Flüssigkeit, so dass ein dichter Abschnitt (3) als der Abschnitt einer Seite ausgebildet wird und ein poröser Abschnitt (5) als der Bereich einer gegenüberliegenden Seite ausgebildet wird.

## Revendications

1. Dispositif de commande (115) qui commande un appareil de mise en forme tridimensionnelle (100), basé sur des données de mise en forme représentant une forme tridimensionnelle d'un objet tridimensionnel mis en forme (10), le dispositif de commande (115) amenant l'appareil de mise en forme tridimensionnelle (100) à exécuter :
un traitement de formation de couche afin de former une couche de céramique, en utilisant un matériau céramique en poudre ;
un traitement d'application de liquide de durcissement afin d'appliquer, à une région cible dans la couche, un liquide de durcissement incluant un agent chélatant ; et
une répétition du traitement de formation de couche et du traitement d'application de liquide de durcissement pour amener l'appareil de mise en forme tridimensionnelle (100) à former l'objet tridimensionnel mis en forme (10) par un produit réactionnel du matériau céramique avec l'agent chélatant dans les régions cibles des couches surimposées l'une sur l'autre,
dans lequel la région cible dans chacune des couches inclut une région d'un côté et une région de côté opposé qui constituent une portion d'un côté et une portion de côté opposé respectivement dans l'objet tridimensionnel mis en forme (10), et
sur la base d'une plage de positions de chacune de la région d'un côté et de la région de côté opposé exprimée par les données de mise en forme, le dispositif de commande (115) amène l'appareil de mise en forme tridimensionnelle (100) à exécuter le traitement d'application de liquide de durcissement de sorte qu'une quantité du liquide de durcissement appliquée à chaque surface unitaire dans la région d'un côté est plus grande qu'une quantité du liquide de durcissement appliquée à chaque surface unitaire dans la région de côté opposé, formant ainsi une portion dense (3) comme portion d'un côté, et formant une portion poreuse (5) comme portion de côté opposé, et
dans lequel la portion dense et la portion poreuse sont formées par un produit réactionnel d'un matériau céramique avec un agent chélatant.

2. Dispositif de commande (115) selon la revendication 1,
dans lequel la région cible dans chacune des couches inclut une région intermédiaire qui constitue une portion intermédiaire (7) entre la portion dense (3) et la portion poreuse (5) dans l'objet tridimensionnel mis en forme (10), et
sur la base d'une plage de positions de chacune de la région d'un côté, de la région de côté opposé, et de la région intermédiaire exprimée par les données de mise en forme, le dispositif de commande (115) amène l'appareil de mise en forme tridimensionnelle (100) à exécuter le traitement d'application de liquide de durcissement de sorte qu'une quantité du liquide de durcissement appliquée à chaque surface unitaire dans la région intermédiaire est inférieure à une quantité du liquide de durcissement appliquée à chaque surface unitaire dans la région d'un côté et est plus grande qu'une quantité du liquide de durcissement appliquée à chaque surface unitaire dans la région de côté opposé, formant ainsi la portion intermédiaire (7) ayant un degré dense supérieur à un degré dense de la portion poreuse (5) et inférieur à un degré dense de la portion dense (3), et
dans lequel la portion intermédiaire présente un degré dense supérieur à un degré dense de la portion poreuse et inférieur à un degré dense de la portion dense.

3. Dispositif de commande selon la revendication 1 ou 2,
dans lequel la portion dense (3) présente un degré dense supérieur ou égal à 80 %, et la portion poreuse (5) présente un degré dense inférieur à 60 %.

4. Dispositif de commande (115) selon l'une quelconque des revendications 1 à 3,
dans lequel le matériau céramique est du phosphate de calcium.

5. Dispositif de commande (115) selon la revendication 4,
dans lequel le phosphate de calcium est n'importe lequel d'un phosphate d'α-tricalcium (α-TCP), d'un phosphate de β-tricalcium (β-TCP), d'un phosphate d'octacalcium (OCP), et d'une carbonate apatite.

6. Dispositif de commande (115) selon la revendication 4 ou 5,
dans lequel l'agent chélatant est un composé organique ayant trois ou plusieurs groupes phosphates ou groupes carboxyle par molécule.

7. Dispositif de commande (115) selon la revendication 6,
dans lequel le composé organique est n'importe lequel de l'acide étidronique, l'acide phytique, et de l'acide citrique.

8. Dispositif de commande (115) selon la revendication 1,
dans lequel l'objet tridimensionnel mis en forme (10) est un os artificiel à charger dans une portion manquante (20a) d'un os vivant (20) afin de former un corps osseux (30) conjointement à l'os vivant (20),
l'os artificiel inclut une surface externe (10a) servant de surface externe du corps osseux, et une surface interne (10b) à localiser à l'intérieur du corps osseux (30), et
la surface externe (10a) est formée par la portion dense (3), et la portion poreuse (5) forme la surface interne (10b).

9. Dispositif de commande (115) selon la revendication 8,
dans lequel la surface interne (10b) formée par la portion poreuse (5) est une surface pour être adjacente à une surface interne incluse dans la portion manquante (20a) et formée par l'os vivant (20).

10. Dispositif de commande (115) selon la revendication 1 ou 9,
dans lequel le traitement d'application de liquide de durcissement inclut en outre :
le fait d'amener l'appareil de mise en forme tridimensionnelle (100) à faire tomber ou à éjecter un liquide d'inclusion de cellules ou un liquide d'inclusion de facteurs de croissance au niveau d'une entièreté ou d'une région prédéterminée de la région cible dans chacune des couches, dans lequel le liquide d'inclusion de cellules inclut des cellules et éventuellement des facteurs de croissance, et le liquide d'inclusion de facteurs de croissance inclut des facteurs de croissance.

11. Procédé de fabrication d'un objet tridimensionnel mis en forme (10), sur la base de données de mise en forme représentant une forme tridimensionnelle d'un objet tridimensionnel mis en forme (10), le procédé comprenant :
une étape de formation de couche afin de former une couche de céramique, en utilisant un matériau céramique en poudre ;
une étape d'application de liquide de durcissement afin d'appliquer, à une région cible dans la couche, un liquide de durcissement incluant un agent chélatant ; et
la répétition de l'étape de formation de couche et de l'étape d'application de liquide de durcissement afin de former l'objet tridimensionnel mis en forme (10) par un produit réactionnel du matériau céramique avec l'agent chélatant dans les régions cibles des couches surimposées l'une sur l'autre,
dans lequel la région cible dans chacune des couches inclut une région d'un côté et une région de côté opposé qui constituent une portion d'un côté et une portion de côté opposé respectivement dans l'objet tridimensionnel mis en forme (10), et
l'étape d'application de liquide de durcissement inclut l'application du liquide de durcissement à la région cible, sur la base d'une plage de positions de chacune de la région d'un côté et de la région de côté opposé exprimée par les données de mise en forme, de sorte qu'une quantité du liquide de durcissement appliquée à chaque surface unitaire dans la région d'un côté est plus grande qu'une quantité du liquide de durcissement appliquée à chaque surface unitaire dans la région de côté opposé, formant ainsi une portion dense (3) comme portion d'un côté, et formant une portion poreuse (5) comme portion de côté opposé.

12. Procédé de fabrication de l'objet tridimensionnel mis en forme (10) selon la revendication 11,
dans lequel la région cible dans chacune des couches inclut une région intermédiaire entre la région d'un côté et la région de côté opposé, et
l'étape d'application de liquide de durcissement inclut l'application du liquide de durcissement à la région cible, sur la base d'une plage de positions de chacune de la région d'un côté, de la région de côté opposé, et de la région intermédiaire exprimée par les données de mise en forme, de sorte qu'une quantité du liquide de durcissement appliquée à chaque surface unitaire dans la région intermédiaire est inférieure à la quantité du liquide de durcissement appliquée à chaque surface unitaire dans la région d'un côté, et est plus grande que la quantité du liquide de durcissement appliquée à chaque surface unitaire dans la région de côté opposé, formant ainsi une portion intermédiaire (7) ayant un degré dense supérieur à un degré dense de la portion poreuse (5) et inférieur à un degré dense de la portion dense (3).

13. Procédé de fabrication d'un objet tridimensionnel mis en forme (10) selon la revendication 11 ou 12, comprenant en outre :
une étape de lavage afin de laver l'objet tridimensionnel mis en forme (10) après formation de l'objet tridimensionnel mis en forme (10).

14. Programme de commande pour commander un appareil de mise en forme tridimensionnelle (100), sur la base des données de mise en forme représentant une forme tridimensionnelle d'un objet tridimensionnel mis en forme (10), le programme de commande amenant l'appareil de mise en forme tridimensionnelle (100) à exécuter :
un traitement de formation de couche afin de former une couche de céramique, en utilisant un matériau céramique en poudre ;
un traitement d'application de liquide de durcissement afin d'appliquer, à une région cible dans la couche, un liquide de durcissement incluant un agent chélatant ; et
un traitement de répétition du traitement de formation de couche et du traitement d'application de liquide de durcissement afin de former l'objet tridimensionnel mis en forme (10) par un produit réactionnel du matériau céramique avec l'agent chélatant dans les régions cibles des couches surimposées l'une sur l'autre,
dans lequel la région cible dans chacune des couches inclut une région d'un côté et une région de côté opposé qui constituent une portion d'un côté et une portion de côté opposé respectivement dans l'objet tridimensionnel mis en forme (10), et
sur la base d'une plage de positions de chacune de la région d'un côté et de la région de côté opposé exprimée par les données de mise en forme, le programme de commande amène l'appareil de mise en forme tridimensionnelle (100) à exécuter le traitement d'application de liquide de durcissement de sorte qu'une quantité du liquide de durcissement appliquée à chaque surface unitaire dans la région d'un côté est plus grande qu'une quantité du liquide de durcissement appliquée à chaque surface unitaire dans la région de côté opposé, formant ainsi une portion dense (3) comme portion d'un côté, et formant une portion poreuse (5) comme portion de côté opposé.
